(19) **Europäisches Patentamt**

**European Patent Office**

**Office européen des brevets**

(11) **EP 0 342 577 B1**

(12) **EUROPEAN PATENT SPECIFICATION**

(45) Date of publication and mention
of the grant of the patent:
**15.03.2000 Bulletin 2000/11**

(51) Int. Cl.[7]: **A61K 31/495**

(21) Application number: **89108729.8**

(22) Date of filing: **12.05.1989**

(54) **Use of dihydropyridine derivatives for the prophylaxis or treatment of cerebral circulation disorders**

Verwendung von Dihydropyridinen zur Vorbeugung und Behandlung cerebraler Durchblutungsstörungen

Utilisation des dérivés de dihydropyridine pour la prévention ou le traitement des maladies de la circulation cérébrale

(84) Designated Contracting States:
**BE CH DE ES FR GB IT LI NL SE**

(30) Priority: **19.05.1988 JP 12261288**

(43) Date of publication of application:
**23.11.1989 Bulletin 1989/47**

(73) Proprietor:
**YOSHITOMI PHARMACEUTICAL INDUSTRIES, LTD.**
**Osaka-shi Osaka (JP)**

(72) Inventors:
• **Uchida, Takeshi**
 **The Green Cross Corp.**
 **Hirakata-shi Osaka 573 (JP)**
• **Ohtaki, Yutaka**
 **The Green Cross Corp.**
 **Hirakata-shi Osaka 573 (JP)**
• **Watanabe, Masahiro**
 **The Green Cross Corp.**
 **Hirakata-shi Osaka 573 (JP)**
• **Ashimori, Atsuyuki**
 **The Green Cross Corp.**
 **Hirakata-shi Osaka 573 (JP)**

• **Fukaya, Chikara**
 **The Green Cross Corp.**
 **Hirakata-shi Osaka 573 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(56) References cited:
**EP-A- 0 094 159**          **EP-A- 0 257 616**
**EP-A- 0 379 737**

• **FOLIA PHARMACOLOGICA JAPONICA, vol. 95, no. 4, 1990, pages 177-184; H. KIDO et**
• **al.: "Angiographic studies on the effects of a novel calcium antagonist AE0047 on cerebral arteries in dogs, with special reference to the preventive effects of AE0047 on cerebral vasoconstriction induced by endothelin"**

Remarks:
The file contains technical information submitted after the application was filed and not included in this specification

**Description**

BACKGROUND OF THE INVENTION

Compounds similar to the dihydropyridine derivatives of the present invention, for example, Nifedipine and Nicardipine are known. It is known that these compounds are useful as an antihypertensive, peripheral and cerebral vasodilator and coronary agent (angina pectoris-treating agent).

The present inventors have conducted various studies and researches for dihydropyridine derivatives having more excellent pharmacological activities (Japanese Patent Application Unexamined Publications (Kokai) Nos. 107975/1988, 112560/1988 and 225356/1988 (equivalent in the content to European Patent Application Publication Nos. 216542, 257616 and 265947 respectively), and Japanese Patent Application No. 3984/1988.

SUMMARY OF THE INVENTION

They studied the pharmacological actions of the compounds thus newly synthesized to find that the below-mentioned particular dihydropyridine derivatives possessed a characteristic property of selectively increasing cerebral blood flow (cerebral hyperkinemic action) as compared with the above-mentioned hitherto-known compounds, and conducted further studies, which resulted in the accomplishment of the present invention.

This invention relates to the use of 2-[p-(4-benzhydrylpiperazino)phenyl]ethyl methyl 2,6-dimethyl-4-(3-nitrophenyl)-1,4-dihydropyridine-3,5-dicarboxylate or 2-(p-(4-benzhydrylpiperazino)phenyl]ethyl methyl 2,6-dimethyl-4-(4-cyano-2-pyridyl)-1,4-dihydropyridine-3,5-dicarboxylate or its acid addition salt for preparing a medicament for selctively increasing cerebral blood flow.

Namely, the above dihydropyridine derivatives and their acid addition salts are characterized in that they have high organ or tissue selectivity in their vasodilative actions and low acute toxicities, and therefore are extremely excellent in safety.

DETAILED DESCRIPTION OF THE INVENTION

The dihydropyridine derivatives and their acid addition salts can be produced by reacting the compound comprising an optional part constituting the dihydropyridine derivatives with the compound comprising the remaining part, particularly by subjecting the compounds to dehydration-cyclization reaction.

Specifically, they are prepared by the production methods disclosed in Japanese Patent Application Unexamined Publications Nos. 107975/1988, 112560/1988, 225356/1988 and 201765/1983 (equivalent in the content to European Patent Application Publication Nos. 216542, 257616, 265947 and 94159 respectively).

The thus-obtained dihydropyridine derivatives and their acid addition salts can be purified to an optional extent by employing known separation-purification means such as concentration, extraction, chromatography, re-precipitation and recrystallization adequately.

Since the dihydropyridine derivatives have basic groups, they can be converted into their acid addition salts by known means. As for the salts, so long as the salts are pharmacologically acceptable non-toxic ones, there is no limitation to them. As such salts, mention may be made of, for example, salts of inorganic acids (e.g. hydrochloride, hydrobromide, phosphate, sulfate) and salts of organic acids (e.g. acetate, succinate, maleate, fumarate, malate, tartarate). These salts can be produced by the per se known methods.

The dihydropyridine derivatives and their acid addition salts may be used singly or two or more species of them may be used in combination.

The dihydropyridine derivatives and their acid addition salts have extremely low toxicity and potent and lasting cerebral vasodilative actions in mammals (e.g. mice, rats, rabbits, dogs, cats, human beings), and thus are useful as cerebral vasodilators and agents for the prophylaxis and therapy of cerebral circulation disorders (e.g. cerebral infarction, transient cerebral ischemic spasm) or aftereffects caused by cerebral circulation disorders (e.g. consciousness disorder, respiratory abnormality, sensory disorder, speech impediment, paralysis, paropsis, blunting of subjective symptom, depression in mentation).

The dihydropyridine derivatives and their acid addition salts employed in the present invention are considerably excellent in specificity, selectivity and duration time in their pharmacological actions on cerebral blood vessels as compared with previously known dihydropyridine derivatives (e.g. Nifedipine, Nicardipine).

The dihydropyridine derivatives and their acid addition salts can be orally or parenterally administered in pharmaceutical composition forms such as powders, granules, tablets, capsules and injections in admixture with pharmaceutically necessary ingredients such as adequate pharmacologically acceptable additives (e.g. carriers, vehicles, diluents). In the above-mentioned pharmaceutical compositions, the dihydropyridine derivatives and their acid addition salts can be contained in an effective amount. While the dosage of the dihydropyridine derivatives varies depending upon the administration route, symptom, weight and age of the patients and the like, for example, the dosage for oral administration to adult patients is preferably 0.05 - 20 mg/kg body weight/day, particularly 0.1 - 4 mg/kg body weight/day, which can be admininstered in once to several times divided doses.

[Examples]

Hereafter, the present invention is explained by illustrating working examples, which should not be construed as being limitative.

Example 1 : Tablets

```
    (1)   Compound 2-2 mentioned below                    10 g

    (2)   Fine particles No. 209 for direct compression  110 g
          (Manufactured by Fuji Chemical Corp.)

              Magnesium metasilicate aluminate   20%

              Corn starch                        30%

              Lactose                            50%

    (3)   Crystalline cellulose                            60 g

    (4)   CMC calcium                                       18 g

    (5)   Magnesium stearate                                2 g
```

(1), (3) and (4) were passed through a sieve of 100 mesh in advance. After (1), (3), (4) and (2) were dried, whereby the water-content was reduced to the specific degree, they were admixed in the above-mentioned weight proportions with the use of a mixer. (5) was added to the powders mixed wholly homogeneously; and the mixture was mixed for a short time (30 seconds). The mixed powders were compressed into tablets weighing 200 mg per tablet.

These tablets may be coated with enteric film-coating agents (e.g. polyvinylacetaldiethylaminoacetate) and edible coloring agents in conventional use.

Example 2 : Capsules

| (1) Compound 2-2 mentioned below | 50 g |
|---|---|
| (2) Lactose | 950 g |
| (3) Magnesium stearate | 20 g |

The above ingredients were weighed and mixed homogeneously and the mixed powders were filled in hard gelatin capsules in an amount of 200 mg per capsule.

Example 3 : Injections

| (1) Compound 2-2 mentioned below | 5 mg |
|---|---|
| (2) Glucose | 100 mg |
| (3) Physiological saline | 10 ml |

The mixture of the above-mentioned ingredients was filtered with a membrane filter and again subjected to sterile filtration. The filtrate was poured, under sterile conditions, into vials, which were filled with nitrogen gas. Thereafter, the vials were sealed to give intravenous injections.

Experimental Example 1 (Selectivity in Cerebral Hyperkinemic Action)

The selectivity in cerebral hyperkinemic action was studied by examining the rates of increase in coronary blood flow amount and cerebral blood flow amount as to various dihydropyridine derivatives.

Various dihydropyridine derivatives were intravenously administered to dogs (weighing 9 - 16 kg, 4 animals per group) at a dose of 1 - 100 µg/kg body weight. At the lapse of 1, 3, 5, 10, 30, 60, 90 and 120 minutes after the intravenous administration, the coronary blood flow amount and cerebral blood flow amount were measured, and the rates of increase in the blood flow amount were estimated.

Coronary blood flow was measured by equipping the left coronary artery circumflex ramus with the probe of the electromagnetic flow meter, and recorded on the polygraph.

Cerebral blood flow was measured by equipping the left vertebral artery with the probe of the electromagnetic flow meter, and recorded continuously on the polygraph.

The results are shown in Table 1. The $ED_{200}$ (µg/kg body weight) indicates the dose at which the rate of increase is 200%.

Table 1

| Compound Dose (μg/kg body weight) | Coronary blood flow amount | | Cerebral blood flow amount | |
|---|---|---|---|---|
| | Increase rate (%) | $ED_{200}$ (μg/kg body weight) | Increase rate (%) | $ED_{200}$ (μg/kg body weight) |
| Nicardipine | | | | |
| 1 | - | | - | |
| 3 | 154 | | 165 | |
| 10 | 206 | 9.7 | 197 | 9.7 |
| 30 | 240 | | 238 | |
| 100 | - | | - | |
| Nifedipine | | | | |
| 1 | - | | - | |
| 3 | 151 | | 140 | |
| 10 | 216 | 9.4 | 212 | 8.7 |
| 30 | 236 | | 264 | |
| 100 | - | | - | |
| Nimodipine | | | | |
| 1 | 125 | | 146 | |
| 3 | 141 | | 167 | |
| 10 | 191 | 16 | 196 | 13 |
| 30 | - | | - | |
| 100 | - | | - | |
| Compound 2-2 (of this Invention) | | | | |
| 1 | - | | - | |
| 3 | - | | - | |
| 10 | 125 | 75 | 197 | 11 |
| 30 | 174 | | 301 | |
| 100 | 207 | | 433 | |
| Compound 1-2 (of this Invention) | | | | |
| 1 | - | | - | |
| 3 | - | | - | |
| 10 | 131 | 48 | 186 | 15 |
| 30 | 156 | | 223 | |
| 100 | 246 | | 385 | |

When the dihydropyridine derivatives and their acid addition salts to be used in the present invention and the hitherto known compounds are compared, as shown in Table 1, it is found that there is little difference in cerebral blood flow, whereas in respect of the rate of increase in coronary blood flow amount, the values of $ED_{200}$ of the dihydropyridine derivatives and their acid addition salts are higher than those of the hitherto-known compounds.

That is, the coronary hyperkinemic actions of the dihydropyridine derivatives and their acid addition salts are not so potent as the cerebral hyperkinemic actions of them.

Accordingly, it is found that the dihydropyridine derivatives and their acid addition salts possess selective cerebral hyperkinemic actions.

Experimental Example 2 (Duration time of Cerebral Hyperkinemic Action)

By examining the rate of increase in cerebral blood flow by various dihydropyridine derivatives with the lapse of time, the duration of cerebral hyperkinemic action was studied.

The experiment was conducted in accordance with the method of Experimental Example 1 except that the dose was exclusively 30 μg/kg body weight. The results are shown in Table 2

Table 2

| | Rate of increase in cerebral blood flow amount | | | | |
|---|---|---|---|---|---|
| | 5 min after administration | 10 min | 30 min | 60 min | 120 min |
| Nicardipine | 237 | 189 | 129 | 109 | - |
| Nifedipine | 263 | 230 | 129 | 100 | - |
| Nimodipine | 163 | 152 | 129 | 113 | - |
| Compound 2-2 | 137 | 170 | 285 | 300 | 237 |
| Compound 1-2 | 183 | 222 | 190 | 151 | 132 |

As shown in Table 2, it is found that the dihydropyridine derivatives and their acid addition salts exhibit stronger cerebral hyperkinemic actions and the actions last longer than the previously-known compounds.

Experimental Example 3 (Acute toxicity)

By using male mice (3 - 5 animals per group) weighing 14 - 16 g, at the age of 10 - 11 weeks, the values of $LD_{50}$ were estimated. The values were all higher than 1400 mg/kg body weight.

Accordingly, the dihydropyridine derivatives and their acid addition salts to be used in the present invention show higher acute toxicity values, and have higher safety than the previously-known compounds.

Experimental Example 4 (Inhibitory effect on symptoms of both common carotid arteries-ligated rats)

1) Used aminal

Spontaneously hypertensive rats (SHR • SR; Japan Charles River Co.) were used for the experiment (9-10 rats per group).

2) Method

The compound 2-2 of the present invention was orally administered forsively to the rats at a dose of 10 ml/kg. To the control group was administered 0.3% Tween® 80 solely at a dose of 10 ml/kg. One hour later, cervical part was incised and both common carotid arteries were ligated with a suture thread almost simultaneously. Two hours after skin suture, observation was made for their neural symptom and/or death. The results are expressed in five-rank scores below.

| Score | Neural symptom |
|---|---|
| 0 | normal |
| 1 | depression of spontaneous movement |

(continued)

| Score | Neural symptom |
|---|---|
| 2 | disappearance of positive response |
| 3 | convulsion |
| 4 | death |

3) Results

Preadministration of the compound of the present invention significantly improved ischemic symptom caused by ligation of both common carotid arteries of spontaneously hypertensive rats.

| Compound of the present invention | Dose (mg/kg) | Average score |
|---|---|---|
| None | - | 2.8 |
| Compound 2-2 | 0.1 | 1.7 |

[Reference Example]

Reference Example

(1) 2-(p-Piperazinophenyl)ethanol

A 200 ml-round bottomed flask was charged with 2-(p-aminophenyl)ethanol (10.153 g, 74.0 mmol) and bis(2-chloro-ethyl)amine hydrochloride (6.605 g, 37.0 mmol), and n-butanol (66 ml) was added thereto. The mixture was heated with stirring in the flask equipped with a condensor for 23.5 hours. After the reaction mixture was cooled to the room temperature, the mixture was poured into water (218 ml) in a 500 ml-beaker. By adding a 15% aqueous solution of sodium hydroxide thereto under ice-cooling, the pH was adjusted 10 - 11, followed by extraction with chloroform (300 ml each time, 5 times). After the chloroform layer was washed with water (150 ml each time, twice) and dried, chloroform was distilled off under reduced pressure. The residue (13.485 g) was separated and purified by column chromatography [silica gel, chloroform-methanol (1:1)] to give 5.996 g of the title piperazine compound (yield: 79%).

$IR\nu_{max}^{KBr}$ cm$^{-1}$ : 3300(OH)
$^1$H-NMR$\delta$ :

7.15-6.95 (2H), 6.95-6.7 (2H), 3.77 (2H, t, J=6Hz), 3.2-2.8 (8H), 2.75 (2H, t, J=6Hz), 2.10 (2H, s)

(2) 2-[p-(4-Benzhydrylpiperazino)phenyl]ethanol

A 100 ml-three-necked flask was charged with the product of (1) (piperazine compound) (5.996 g, 29.1 mmol), and the flask was equipped with a condensor in the center and septum rubbers in the left and right necks. In the flask was added 33 ml of dimethylformamide to dissolve the content, and thereafter potassium carbonate (8.034 g, 58.1 mmol) and bromodiphenylmethane (7.543 g, 30.5 mmol) were added. The mixture was stirred at room temperature for 2 hours. The reaction mixture was added to water (80 ml) in a 200 ml-flask, and the mixture was extracted with diethyl ether (100 ml each time, four times). The ether layer was washed with water (40 ml each time, twice), and dried. After ether was distilled off under reduced pressure, the residue (10.616 g) was separated and purified by column chromatography [silica gel, ethyl acetate-n-hexane (1:1)] to give 6.188 g of the title benzhydrylpiperazine compound (yield 57%).

$$IR\nu_{max}^{CDCl_3} cm^{-1} : 3600(OH)$$

$^1$H-NMRδ :

7.55-6.9 (12H), 6.9-6.55 (2H), 4.23 (1H, s), 3.72 (2H, t, J=6Hz), 3.3-2.95 (4H), 2.95-2.35 (6H), 1.71 (1H, s)

(3) <u>2-[p-(4-Benzhydrylpiperazino)phenyl]ethyl acetoacetate</u>

A 200 ml-three-necked flask was charged with the product of (2) (Benzhydrylpiperazine compound) (5.970 g, 16.0 mmol), and the flask was equipped with an air condensor in the center, a thermometer in one of the remaining necks and a septum rubber in the other. After diethyl ether (50 ml) was added in the flask to dissolve the content, and the mixture was cooled (with ice-sodium chloride) to -13 to -12°C, diketene (1.531 g, 18.2 mmol) and dimethylaminopyridine (7 mg, 0.057 mmol) were added thereto. The mixture was stirred at the same temperature for 30 minutes and at room temperature for 17.5 hours. The reaction mixture was ice-cooled, and a 0.1% aqueous solution of sodium hydroxide (65 ml) was added. After washed, the diethyl ether layer was separated. The water layer was extracted with ether (120 ml each time, three times). The ether layers were combined, washed with a 0.1% aqueous solution of sodium hydroxide (65 ml each time, twice) and water (50 ml each time, twice) and dried. The solvent was distilled off under reduced pressure to give 7.364 g of the title acetoacetate quantitatively.

$IR\nu_{max}^{neat}$ cm$^{-1}$ : 1740(C=O), 1720(C=O)
$^1$H-NMRδ :

7.6-6.9 (12H), 6.9-6.65 (2H), 4.29 (2H, t, J=6.5Hz), 4.24 (1H, s), 3.39 (2H, s), 3.35-3.0 (4H), 2.85 (2H, t, J=6.5Hz), 2.7-2.4 (4H), 2.18 (3H, s)

(4) <u>2-[p-(4-Benzhydrylpiperazino)phenyl]ethyl methyl 2,6-dimethyl-4-(4-cyano-2-pyridyl)-1,4-dihydropyridine-3,5-dicarboxylate (Compound 1-1) and its mono-hydrochloride (Compound 1-2)</u>

A 50 ml-round bottomed flask was charged with 4-cyano-2-pyridinealdehyde (684 mg, 5.18 mmol), the objective compound of (3) (2.363 g, 5.18 mmol) and methyl 3-aminocrotonate (614 mg, 5.18 mmol), whereto isopropanol (7 ml) was added.
The mixture was stirred at 40 - 50°C for 26 hours in the flask equipped with a condensor. After the reaction solvent was distilled off, the residue (3.539 g) was subjected to column chromatography [silica gel, ethyl acetate-n-hexane (3:1) and (2:1)] for separation to give the crude product. The obtained crude product was recrystallized from chloroform-methanol to give 1.649 g of Compound 1-1 (yield 48%).

m.p. (when crystallized from chloroform-methanol) : 218 - 220°C
$IR\nu_{max}^{KBr}$ cm$^{-1}$ : 2225(CN), 1700(C=O), 1665(C=O)
$^1$H-NMRδ :

8.65-8.45 (1H), 7.6-6.65 (17H), 5.16 (1H, s), 4.4-4.0 (3H), 3.59 (3H, s), 3.3-2.95 (4H), 2.95-2.45 (6H), 2.25, 2.22 (each 3H, s)

Compound 1-1 (1.092 g, 1.64 mmol) was put in a 100 ml-three-necked flask, which was equipped with an air condensor in the center, and septum rubbers in the right and left necks. Methylene chloride (21 ml) was added in the flask to dissolve the content, followed by addition of a solution of hydrogen chloride in dioxane (1.20 N, 1.363 ml). The mixture was stirred at room temperature for 5 hours. After the reaction solvent was distilled off under reduced pressure, ethanol (15 ml) was added to the residue. The solvent was distilled off under reduced pressure to give about 1.15 g of Compound 1-2.

$IR\nu_{max}^{KBr}$ cm$^{-1}$ : 2225(CN), 1680(C=O)

$$^{1}\text{H-NMR}\delta_{DMSO-d_6 + CDCl_3 \ (2:1.5)} :$$

8.74 (1H, s), 8.57 (1H, d, J=5Hz), 7.55-7.1 (12H), 7.1-6.6 (4H), 5.05 (1H, s), 4.35-3.95 (2H), 3.75-2.6 (14H), 2.25, 2.23 (each 3H, s)

(5) 2-[p-(4-Benzhydrylpiperazino)phenyl]ethyl methyl 2,6-dimethyl-4-(3-nitrophenyl)-1,4-dihydropyridine-3,5-dicarboxylate (Compound 2-1) and its mono-hydrochloride (Compound 2-2)

A 100 ml-round bottomed flask was charged with 3-nitro-benzaldehyde (1.144 g, 7.57 mmol), the objective compound of (3) (3.464 g, 7.59 mmol) and methyl 3-aminocrotonate (873 mg, 7.58 mmol), whereto isopropanol (12 ml) was added. The mixture was heated under reflux for 16 hours in the flask. equipped with a condensor. After the reaction solvent was distilled off under reduced pressure, the residue was subjected to column chromatography [silica gel, chloroform-methanol (45:1)] and column chromatography [silica gel: ethyl acetate-n-hexane (2:3)] for separation to give the crude product, which was purified by high performance liquid chromatography to give 2.503 g of the above titled Compound 2-1 (yield: 48%).

IR$\nu_{max}^{KBr}$ cm$^{-1}$ : 1680(C=O), 1520(NO$_2$)
$^1$H-NMR$\delta$ :

8.06 (1H, t, J=2Hz), 7.97 (1H, ddd, J=8; 2; 1Hz), 7.1-7.6 (12H), 7.03 (2H, d, J=8.6Hz), 6.80 (2H, d, J=8.6Hz), 6.02, (1H, s), 5.07 (1H, s), 4.26 (1H, s), 4.22 (2H, t, J=7Hz), 3.64 (3H, s), 3.15 (4H, dd, J=5; 4.7Hz), 2.81 (2H, t, J=7Hz), 2.55 (4H, dd, J=5; 4.7Hz), 2.33, 2.28 (each 3H, s)

This Compound 2-1 (2.124 g, 3.16 mmol) was put in a 200 ml-round bottomed flask, which was equipped with a septum rubber. Methylene chloride (100 ml) was added in the flask to dissolve the content, whereto a dioxane solution of hydrogen chloride (1.20 N, 2.64 ml) was added. The mixture was stirred at room temperature for 30 minutes. After the reaction solvent was distilled off under reduced pressure to give about 2.22 g of the title Compound 2-2.

IR$\nu_{max}^{KBr}$ cm$^{-1}$ : 2450(>N$^+$H$^-$Cl), 1680(C=O), 1520(NO$_2$)
$^1$H-NMR$\delta$ :

9.17 (1H, s), 8.1 -7.9, 7.5-7.3 (14H), 7.08 (2H, d, J=8Hz), 6.85 (2H, d, J=8Hz), 5.73 (1H, d, J=9Hz), 4.97 (1H, s), 4.14 (2H, t, J=6Hz), 3.8-3.55 (7H), 3.23 (4H, brs), 2.77 (2H, t, J=6Hz), 2.30, 2.26 (each 3H, s)

## Claims

### Claim for the following Contracting States : BE, CH, LI, DE, FR, GB, IT, NL, SE

1.    Use of 2-[p-(4-benzhydrylpiperazino)phenyl]ethyl methyl 2,6-dimethyl-4-(3-nitrophenyl)-1,4-dihydropyridine-3,5-dicarboxylate or 2-[p-(4-benzhydrylpiperazino)phenyl]ethyl methyl 2,6-dimethyl-4-(4-cyano-2-pyridyl)-1,4-dihydropyridine-3,5-dicarboxylate or its acid addition salt for preparing a medicament for selectively increasing cerebral blood flow.

### Claim for the following Contracting State : ES

1.    Method to manufacture a medicament for selectively increasing cerebral blood flow characterized in the use of 2-[p-(4-benzhydrylpiperazino)phenyl]ethyl methyl 2,6-dimethyl-4-(3-nitrophenyl)-1,4-dihydropyridine-3,5-dicarboxylate or 2-[p-(4-benzhydrylpiperazino)phenyl]ethyl methyl 2,6-dimethyl-4-(4-cyano-2-pyridyl)-1,4-dihydropyridine-3,5-dicarboxylate or its acid addition salt.

## Patentansprüche

### Patentanspruch für folgende Vertragsstaaten : BE, CH, LI, DE, FR, GB, IT, NL, SE

1.    Verwendung von 2-[p-(4-Benzhydrylpiperazino)phenyl]ethylmethyl-2,6-dimethyl-4-(3-nitrophenyl)-1,4-dihydropyridin-3,5-dicarboxylat oder 2-[p-(4-Benzhydrylpiperazino)phenyl]ethylmethyl-2,6-dimethyl-4-(4-cyan-2-pyridyl)-1,4-dihydropyridin-3,5-dicarboxylat oder seines Säureadditionssalzes zur Herstellung eines Medikaments zur selektiven Erhöhung des cerebralen Blutstroms.

### Patentanspruch für folgenden Vertragsstaat : ES

1.    Verfahren zur Herstellung eines Medikaments zur selektiven Erhöhung des cerebralen Blutstroms, gekennzeichnet durch die Verwendung von 2-[p-(4-Benzhydrylpiperazino)phenyl]ethylmethyl-2,6-dimethyl-4-(3-nitrophenyl)-1,4-dihydropyridin-3,5-dicarboxylat oder 2-[p-(4-Benzhydrylpiperazino)phenyl]ethylmethyl-2,6-dimethyl-4-(4-cyan-2-pyridyl)-1,4-dihydropyridin-3,5-dicarboxylat oder seines Säureadditionssalzes.

**Revendications**

**Revendication pour les Etats contractants suivants : BE, CH, LI, DE, FR, GB, IT, NL, SE**

**1.** Utilisation de 2,6-dlméthyl-4-(3-nitrophényl)-1,4-dihydropyridine-3,5-dicarboxylate de 2-[p-(4-benzhydrylpipérazino)phényl]éthyl méthyle ou de 2,6-diméthyl-4-(4-cyano-2-pyridyl)-1,4-dihydropyridine-3,5-dicarboxylate de 2-[p-(4-benzhydrylpipérazino)phényl]éthyl méthyle ou de leurs sels d'addition d'acides pour la préparation d'un médicament visant à accroître sélectivement l'irrigation sanguine cérébrale.

**Revendication pour l'Etat contractant suivant : ES**

**1.** Procédé de fabrication d'un médicament pour augmenter sélectivement de l'irrigation sanguine cérébrale caractérisé par l'utilisation de 2,6-diméthyl-4-(3-nitrophényl)-1,4-dihydropyridine-3,5-dicarboxylate de 2-[p-(4-benzhydrylpipérazino)phényl]éthyl méthyle ou de 2,6-diméthyl-4-(4-cyano-2-pyridyl)-1,4-dihydropyridine-3,5-dicarboxylate de. 2-[p-(4-benzhydrylpipérazino)phényl]éthyl méthyle ou de leurs sels d'addition d'acides.